# EUROPÄISCHE PATENTANMELDUNG

(11) **EP 3 756 554 A1**
(43) Veröffentlichungstag der Anmeldung: **30.12.2020**
(21) Anmeldenummer: 19000544.7
(22) Anmeldetag: 25.05.2019
(51) Int. Cl.: A61B 10/02, A61B 17/3209, A61B 17/3211

(54) **EXCISIONSSKALPELL**

(71) Anmelder: Barth, Georg, 81679 München (DE)
(72) Erfinder: Barth, Georg, 81679 München (DE)

(57) **Zusammenfassung**

Die Erfindung betrifft ein 1. Excisionsskalpell (1) zur Gewinnung von Gewebeproben für histologische Untersuchungen mit einem Schneideteil (2) und einem Griffteil (3), dadurch gekennzeichnet, dass das Schneideteil (2) zumindest zwei mit einander verbundene, flache Schenkel (4) aufweist, die an einer Vorderkante (5) zumindest bereichsweise je eine Schneide(6) aufweisen und dass einer der Schenkel (4) mit dem Griffteil (3) verbunden ist. Die Erfindung betrifft auch eine Excisionseinheit mit einem Excisionsskalpell (1) und einem Behalterteil (31), in welches das Excisionsskalpell (1) nach Art eines Stopfens einsetzbar ist.

## Beschreibung

Die Erfindung betrifft ein Excisionsskalpell zur Gewinnung von Gewebeproben für histologische Untersuchungen gemäß dem Oberbegriff des Anspruchs 1 sowie eine Excisionseinheit mit einem Excisionsskalpell gemäß dem Oberbegriff des Anspruchs 19.

Die Erfindung betrifft ein Gebiet der Medizin, insbesondere den Bereich medizinischer Instrumente oder Werkzeuge für Biopsien, das heißt zur Gewinnung von Gewebeproben der Haut, Schleimhaut, Knorpel oder anderer relevanter Gewebe. Derartige Gewebeproben werden üblicherweise von kleinen Arealen der Haut oder anderer Gewebe gewonnen, die Auffälligkeiten zeigen, deren Natur durch eine histologische Untersuchung geklärt werden soll. Bei den Gewebeproben können selbstverständlich auch gesunde Bereiche mit erfasst werden. Biopsien der Haut oder anderer Gewebe bei Menschen oder Tieren gehören zu den Routineverrichtungen in der dermatologischen Praxis und bestehen in dem Ausschneiden und Entnehmen von kleinen Hautbereichen eines Patienten.

Zur Gewinnung von der Gewebeproben sind verschiedene Methoden und Instrumente im Einsatz. Bei einem bekannten Verfahren, wie es beispielsweise in DE 34 06 961 A1 beschrieben ist, wird eine Biopsiestanzvorrichtung verwendet, die im Wesentlichen aus einem länglichen Zylinder besteht. Ein Ende des Zylinders dient als Handhabe und das andere Ende weist eine zylindrische Schneide auf. Bei Benutzung wird die zylindrische Schneide gegen die Haut des Patienten gedrückt und dringt in diese bis zu einer bestimmten Tiefe ein. Danach wird die Biopsiestanze entfernt und die Basis des ausgestanzten, kreisförmigen Bereichs wird mit einem Skalpell oder einer Schere durchtrennt und die Gewebeprobe mit einer Pinzette oder Zange entfernt. Anschließend wird die hierbei entstandene Wunde mit einem oder zwei Nähten geschlossen um eine Blutung zu unterbinden. Die Gewebeprobe wird danach in einem mit Formalin gefüllten Behälter zur Untersuchung in ein Labor geschickt.

Ähnlich aufgebaute Biopsiestanzen sind in DE 44 18 676 A1 und DE 10 2009 018 940 A1 beschrieben. Bei diesen Biopsiestanzen ist der Schneidenbereich nicht kreisförmig, sondern länglich oval oder lanzettförmig ausgebildet. Dies ergibt eine entsprechend geformte Wunde, die besser zu versorgen ist als eine kreisförmige, was auch ein besseres Narbenbild ergibt, insbesondere wenn die Kontur der Schneide gezackt ist, wie in DE 10 2009 018 940 A1 ausgeführt ist.

Die Verwendung von Biopsiestanzen hat zahlreiche Nachteile. Zur Durchführung der Biopsie sind neben der Biopsiestanze mehrere chirurgische Instrumente erforderlich, wie Scheren, Pinzetten, Nadeln und Nadelhalter sowie Nahtmaterial. Der Vorgang ist somit zeitraubend und kostenintensiv. Zudem kommt es vor, dass die Gewebeprobe im Rohr der Biopsiestanze festsitzt und mittels einer Nadel extrahiert werden muss, bevor sie in den Versandbehälter überführt werden kann. Speziell für Biopsien ausgebildete Behälter sind beispielsweise in DE 10 2010 027 488 A1 und WO 2013/023075 A1 beschrieben. In DE 10 2005 034 217 A1 ist ein System mit einem Abstrichinstrument für die Entnahme von Zellen sowie eine Aufnahmevorrichtung hierfür beschrieben. Die Aufnahmevorrichtung ist als rohrförmiger Behälter ausgebildet, in den das Abstrichinstrument mit den an ihm anhaftenden Zellen einführbar ist. Durch das Einführen des Abstrichinstruments entsteht eine geschlossene Kammer in der das Präparat geschützt ist und aufbewahrt oder transportiert werden kann.

Manche Ärzte verwenden Küretten für Biopsien. Um hinreichend dickes Biopsiematerial zu gewinnen, muss hierbei relativ tief in die Dermis eingedrungen werden. Dies führt zu schalenförmig eingetieften Gewebedefekten mit dem Risiko von Blutungen und von unästhetischen Narbenbitdungen. Dies ist besonders nachteilig im Gesichtsbereich, in dem die meisten Hautkrebsbildungen oder andere unerwünschte Veränderungen auftreten. Bei der Verwendung von Küretten ist die Gewebeprobe oft fragmentiert und bietet Probleme bei der histologischen Untersuchung, weil die Küretten nicht hinreichend scharf sind. Eine Kürette zur Entfernung von größeren Gewebeteilen aus dem Kniebereich eines Patienten ist z.B. In US 2 521 161 beschrieben. Sie ist schon wegen ihrer Größe nicht für Feinbiopsien im Gesicht geeignet. In US 2017/0265887 A1 ist eine verstellbare Kürette gezeigt, die ein Griffteil und ein aus einem flexiblen Bandmaterial hergestelltes Schneideteil aufweist. Ein Ende des Bandmaterials ist im Inneren des Griffteils fest fixiert. Das andere Ende des Bandmaterials ist mit einem Schieber verbunden, der im Inneren des Griffteils verschieblich aber in bestimmten Positionen arretierbar gelagert ist. Das Bandmaterial tritt am distalen Ende des Griffteils aus und bildet hier eine Schlaufe, deren Größe durch Verschieben des Schiebers änderbar ist. Die Schlaufe bildet hierbei den Schneidebereich der Kürette, Bei Benutzung der Kürette sind beide Schenkel des Schneideteils im Griffteil fixiert.

Die Verwendung von einfachen Skalpellen zur Gewinnung von Biopsien ist nicht üblich, da vom Operateur mehrere Schnitte nacheinander ausgeführt werden müssen um beide Seiten und den Untergrund der Gewebeprobe freigelegt werden müssen. Dies ist zeitraubend und ergibt öfters unregelmäßig geformte Präparate.

Schließlich sind Hautbiopsien ein Hauptgrund für Verzögerungen bei dermatologischen Konsultationen. Eine Biopsie beansprucht ungefähr 15 bis 30 Minuten, während derer alles benötigte Material bereitgestellt und eingesetzt wird um die Prozedur durchzuführen. Deswegen wird öfters auf diese Diagnosemöglichkeit verzichtet, obwohl sie der Sache dienlich wäre. Solche Versäumnisse sind in Ländern, wie den USA, ein häufiger Grund für Klagen vor Gericht.

Der Erfindung liegt daher die Aufgabe zugrunde, ein Biopsiewerkzeug der eingangs genannten Art zu schaffen, mit dem eine schnelle und kostengünstige Biopsie zur Gewinnung von Gewebeproben der Haut möglich ist. Eine weitere Aufgabe der Erfindung besteht darin, eine Excisionseinheit anzugeben, die in Verbindung mit dem Biopsiewerkzeug eine einfache Handhabung der gewonnenen Gewebeproben ermöglicht.

Die Lösung dieser Aufgabe erfolgt gemäß dem kennzeichnenden Teil des Anspruchs 1 dadurch, dass das Schneideteil mindestens zwei mit einander verbundene, flache Schenkel aufweist, die an einer Vorderkante zumindest bereichsweise je eine Schneide aufweisen und dass nur einer der Schenkel mit dem Griffteil fest verbunden ist.

In einer bevorzugten Ausführung des erfindungsgemäßen Excisionsskalpells sind zwei verschieden lange Schenkel unter einem Winkel zueinander angeordnet, wobei der längere Schenkel im Griffteil fixiert ist. und Die Schenkel bilden hierdurch eine V-Form im distalen Bereich des Excisionsskalpells, wobei die beiden Schneiden im Spitzenbereich des V ausgebildet sind und dort lückenlos ineinander übergehen. Einer oder beide Schenkel des Schneideteils können in Ausführung der Erfindung geknickt sein, so dass ein "V" gebildet ist, dessen obere Endbereiche durch bevorzugt parallele Teile der Schenkel verlängert sind.

Ein mit einem derartigen Excisionsskalpell ausgeführter Schnitt durch ein Hautareal ergibt somit eine längliche, fadenförmige Gewebeprobe mit einem dreieckigen Querschnitt, deren Länge vom Operateur nach Belieben bestimmt werden kann. Mit einem einzigen Instrument können somit lückenlose Gewebeproben über die gesamte Erstreckung eines zu untersuchenden Hautareals gewonnen werden. Diese Möglichkeit ist bei einer herkömmlichen Biopsiestanze nicht gegeben, da die Größe des ausgestanzten Bereichs über den Durchmesser des Schneideteils festgelegt ist.

In einer weiteren, besonders bevorzugten Ausführungsform weist das Schneideteil eine U-Form oder Tropfenform mit zwei im Wesentlichen geraden Schenkeln und einem die Schenkel verbindenden, gebogenen Bereich oder Schenkel auf. Die beiden geraden Schenkel des "U" sind verschieden lang. Der längere der beiden Schenkel ist im Griffteil fest verankert, während der andere Schenkel frei liegt. Bei dieser Bauform ist nicht nur der gebogene Bereich eine Schneide versehen, sondern auch die an ihn anschließenden geraden Bereiche der beiden Schenkel des Schneideteils des Excisionsskalpells sind als Schneiden ausgebildet.

Diese Ausführung des Excisionsskalpells ergibt eine Gewebeprobe mit einem Querschnitt in U-Form, mit ebenfalls frei wählbarer Länge. Die Breite der generell fadenförmigen Gewebeprobe ist durch den Abstand der beiden Schenkel festgelegt, der bevorzugt im Bereich von 0,5 bis 5 mm liegt, wobei der Bereich von 1 bis 2 mm besonders bevorzugt ist.

In einer vorteilhaften Abwandlung der zuvor beschriebenen Bauformen des erfindungsgemäßen Excisionsskalpells kann der mit Schneiden versehene Bereich des Schneideteils auch unter einem Winkel zum Griffteil angeordnet sein, so dass sich eine Anordnung in Form eines Hockeyschlägers ergibt. Hiermit ist es möglich Gewebeproben auch unterhalb einer Hautschicht zu gewinnen.

Ein erfindungsgemäßes Excisionsskalpell kann auch derart ausgebildet sein, dass das Schneideteil einen geraden Schenkel und einen gebogenen Schenkel von verschiedener Länge aufweist, deren Schneiden lückenlos ineinander übergehen, wobei nur der, längere Schenkel mit dem Griffteil verbunden ist. Diese Bauart ergibt somit eine J-förmige Anordnung der Schneiden des Schneideteils, welche die Entnahme der erhaltenen Gewebeprobe erleichtert. Alternativ hierzu kann auch nur der gebogene Schenkel des Schneideteils mit dem Griffteil verbunden sein, während der gerade Schenkel frei liegt.

Eine erleichterte Entnahme der Probe ist bei sämtlichen Ausführungen der Erfindung möglich, wenn die Schenkel oder gebogenen Bereiche des Schneideteils sich entgegen der Schnittrichtung trichterförmig erweitern. Die Schnittrichtung ist selbstverständlich durch die Anordnung der Schneiden am Schneideteil vorgegeben.

In einer besonderen Ausführungsform des erfindungsgemäßen Excisionsskalpells weist das Schneideteil einen zentralen geraden Schenkel auf, der mit dem Griffteil fest verbunden ist. Am distalen Ende dieses Schenkels, d.h. an dessen Spitzenbereich ist nach jeder Seite hin je ein weiterer Schenkel mit je einer Schneide angeordnet. Diese beiden Schenkel sind gebogen oder weisen in ihrem Verlauf einen Knick auf, so dass das Schneideteil insgesamt die Form eines Schiffsankers besitzt. Die beiden zusätzlichen Schenkel weisen bevorzugt dieselben Dimensionen und Formen auf, was jedoch nicht zwingend ist. Sie können selbstverständlich auch verschieden groß und verschieden geformt sein. Mit einer derartigen Bauform des Schneideteils können mit einem Schneidevorgang gleichzeitig zwei getrennte Gewebeproben aus demselben Bereich eines Gewebes gewonnen werden.

In einer weiteren vorteilhaften Ausführungsform der Erfindung ist am Griffteil oder am Schneideteil ein seitliches Ansatzteil angeordnet oder ausgebildet, wobei das Ansatzteil in seinem freien Endbereich einen Durchbruch oder eine durchgehende Bohrung aufweist. Dieser Ansatz erstreckt sich rechtwinklig zur Schnittrichtung und zur Längsachse des bevorzugt stabförmigen Griffteils. Die im Ansatzteil ausgebildete Bohrung dient zur Fixierung des Excisionsskalpells auf einem Hautareal mittels einer Nadel. Das Excisionsskalpell kann somit nach Art eines Zirkels um eine durch die Nadel definierte Drehachse geführt werden, wodurch eine kreisbogenförmige Gewebeprobe erzielbar ist.

Das Schneideteil ist in Ausführung der Erfindung bevorzugt aus einem metallischen oder keramischen Werkstoff hergestellt, der zur Ausbildung einer besonders dünnen und scharfen Schneidkante geeignet ist. In Frage kommen hierfür insbesondere Edelstähle oder Keramiken, wie Zirkon oder Aluminiumoxid, die unter anderem zur Herstellung von chirurgischen Instrumenten oder von Schneidwaren für den Haushalt verwendet werden. Besonders bevorzugt ist das Schneideteil aus einem flexiblen, biegbaren Band hergestellt, von dem zumindest eine Seitenkante als eine Schneide ausgebildet ist. Solche flexiblen, biegbare Bänder, die aus rostfreiem Stahl hergestellt sind, sind beispielsweise aus handelsüblichen Rasierapparaten für die Nassrasur mit mehreren, gestaffelten Klingen bekannt.

Die Erfindung umfasst auch Ausführungsformen bei denen der Abstand zwischen zwei Schenkeln veränderbar ist. Hierbei findet beispielsweise eine Stellschraube Verwendung, die. in einem Schenkel gelagert ist und gegen einen gegenüberliegenden Schenkel Druck ausüben kann. Es versteht sich, dass das Material des Schneideteils hierfür ausreichend flexibel sein muss, um ein Verbiegen zuzulassen.

Das Griffteil eines Excisionsskalpells gemäß der Erfindung ist generell stabförmig und in einer bevorzugten Bauform zumindest bereichsweise abgeflacht und/oder an der Oberfläche strukturiert. Die Strukturierung kann beispielsweise in rautenförmigen Erhebungen und Vertiefungen bestehen oder in einer Riffelung. Die beiden Formgebungen dienen zur Erhöhung der Griffigkeit und damit der sicheren Führung des Excisionsskalpell durch den Operateur. Als Material für das Griffteil sind Kunststoffe oder Metalle, wie Edelstahl, oder Metalllegierungen, eventuell mit einer Chrombeschichtung, geeignet.

Eine vorteilhafte Ausgestaltung des Excisionsskalpells kann darin bestehen, dass das Griffteil Mittel zum lösbaren Fixieren deslängeren einen Schenkels des Schneideteils aufweist. Dies gestattet es, nach Belieben verschieden geformte Schneideteile mit einem Griffteil einzusetzen. Die Fixierung des Schneideteils kann beispielsweise durch Pressung der Schenkel des Schneideteils zwischen Klemmbacken oder durch Verschraubung erfolgen.

Alternativ kann das Schneideteil mit dem Griffteil fest verbunden sein, was sich besonders bei einer Ausführungsform als Einmal-Instrument anbietet. Die Verbindung der beide Bauteile erfolgt bevorzugt durch Eingießen, wofür Kunststoffe als Material für das Griffteil besonders geeignet sind, Zur sicheren Fixierung des Schneideteils im Griffteil ist es von Vorteil, wenn die einzugießenden Bereiche des Schneideteils mit Durchbrüchen versehen sind, durch die das Eingußmaterial hindurchtreten kann.

In einer besonders bevorzugten Ausführungsform weist das Griffteil in dem dem Schneideteil benachbarten Bereich eine Sollbruchstelle auf, die beispielsweise durch eine Kerbe gebildet sein kann. Hierdurch wird das Material des Griffteils derart geschwächt, dass durch Knicken ein Bruch des Griffteils erfolgen kann. Eine solche Kerbe ist bevorzugt so angeordnet, dass das Schneideteil mit einem relativ kurzen Teil des Griffteils von einem längeren Teil abgetrennt werden kann. Dies ermöglicht es, nach erfolgter Biopsie den Schneideteil mit einer daran anhaftenden Gewebeprobe und dem kurzen Stück des Griffteils in einen Behälter von kleinem Volumen zu überführen.

In einer alternativen Ausführungsform ist das Griffteil zweiteilig ausgebildet, wobei die beiden Teile lösbar miteinander verbunden sind. Die Verbindung der beiden Teile des Griffteils kann beispielsweise durch Ineinanderstecken erfolgen, wofür die zu verbindenden Enden der beiden Teile komplementär zueinander ausgebildet sind.

In Ausführung der Erfindung kann das Griffteil nach Belieben mit zusätzlichen Funktionen ausgestattet sein, wozu beispielsweise auch eine Beleuchtungseinrichtung für den Eingriffsbereich gehört.

Zur Lösung der eingangs genannten Aufgabe ist im Hinblick auf die Excisionseinheit nach einem der Ansprüche 19 bis 21 ist vorgesehen, dass die Excisionseinheit ein Behälterteil aufweist, welches mit dem Excisionsskalpell gemäß der Erfindung in lösbarer Weise verbindbar ist.

In einer bevorzugten Ausführungsform der Excisionseinheit ist das Behälterteil rohrförmig mit einem verschlossenen Boden und mit einer dem Boden gegenüberliegenden Öffnung ausgebildet, in welche das Excisionsskalpell einführbar und fixierbar ist. Das Griffteil des Excisionsskalpells ist hierfür bevorzugt derart ausgebildet, das es die Öffnung des Behälterteils als Deckel verschließt. In einer bevorzugten Ausführungsform weist das Griffteil einen verdickten Bereich auf, dessen Form und Dimensionen derart die die Form und Größe der Öffnung des Behälterteils angepasst ist, dass das Griffteil mit einer daran anhaftenden Gewebeprobe nach Art eines abdichtenden Stopfens in das Behälterteil einsetzbar ist. Dies ermöglicht es, die Gewebeprobe in das mit Formalin gefüllte Behälterteil einzuführen oder zu entnehmen, ohne dass die Verwendung eines weiteren Werkzeugs erforderlich ist. Zugleich ist die Entnahme der Gewebeprobe aus dem Behälter in gleicher Weise erleichtert.

In einer besonders bevorzugten Ausführungsform der Erfindung ist das Griffteil zweiteilig ausgebildet, wobei ein erster, relativ kurzer Teil mit dem Schneideteil fest verbunden ist und ein längerer, den eigentlichen Griff bildender zweiter Teil lösbar mit dem ersten Teil verbunden ist. Diese Bauform gestattet es, nach erfolgter Biopsie den ersten Teil mit dem Schneideteil und der daran anhaftenden Gewebeprobe vom zweiten Teil abzutrennen und in einen handelsüblichen, bevorzugt mit Formalin gefüllten und verschließbaren Transportbehälter zu überführen.

Das Excisionsskalpell gemäß der Erfindung behebt zum großen Teil die eingangs geschilderten Nachteile der Excisionswerkzeuge gemäß dem Stand der Technik. Es erlaubt die schnelle und kostengünstige Gewinnung von Gewebeproben der Haut mit einem geringen apparativen Aufwand schon beim ersten Besuch des Patienten in der dermatologischen Praxis. Dies bringt eine Ersparnis an Zeit und vermindert Verzögerungen bei der Diagnose. Zudem erlaubt es eine einfache Festlegung der Dicke der abgelösten Hautschicht bei der Biopsie, da sich die Eindringtiefe durch den auf das Instrument ausgeübten Druck des Operateurs bestimmen lässt. Dadurch sind epidermale, dermale, subkutane oder tiefere Gewebeproben ohne weiteres zu erhalten. Die Schnittrichtung, d.h. die Vorschubrichtung bei der Durchführung der Biopsie, ist durch die Position der Schneiden des Excisionsskalpells vorgegeben, wobei ein ziehender Schnitt bevorzugt ist, da er eine bessere Kontrolle der Eindringtiefe und damit der Dicke der Gewebeprobe gestattet. Durch die offene Bauform des erfindungsgemäßen Excisionsskalpells ist der Eingriffsbereich für den Operateur besser zu übersehen, als dies mit Küretten oder Excisionsstanzen möglich ist.

Die mit dem erfindungsgemäßen Excisionsskalpell verursachten Wunden haben eine längliche Streifenform, deren Ränder leicht mit einem Klammerpflaster zusammenziehbar sind und die mit einem Kompressionsverband kurzzeitig versorgt werden können. Die danach gebildete Narbe ist kaum sichtbar und somit wesentlich ästhetischer, als ein durch Küretten oder Excisionsstanzen verursachtes Ergebnis. Dies ist besonders vorteilhaft, wenn der Eingriff im Gesicht durchgeführt wurde.

Die Erfindung wird anschließend an Hand von Ausführungsbeispielen, die in den Figuren dargestellt sind, noch näher erläutert. Es zeigen:
Fig. 1 ein erstes Ausführungsbeispiel eines Excisionsskalpells gemäß der Erfindung;
Fig. 2 eine Detailansicht eines Schneideteils gemäß einem ersten Ausführungsbeispiel;
Fig. 3 eine Detailansicht eines weiteren Schneideteils;
Fig. 4 eine Detailansicht eines dritten Schneideteils mit einem Ansatzteil;
Fig. 5 eine Detailansicht eines weiteren alternativen Schneideteils;
Fig.6 eine Detailansicht des vorderen Bereiches eines Griffteils gemäß der Erfindung;
Fig. 7 eine Detailansicht eines alternativen Schneideteils in Ankerform;
Fig. 8 eine Excisionseinheit gemäß der Erfindung in einem zusammengesteckten Zustand;

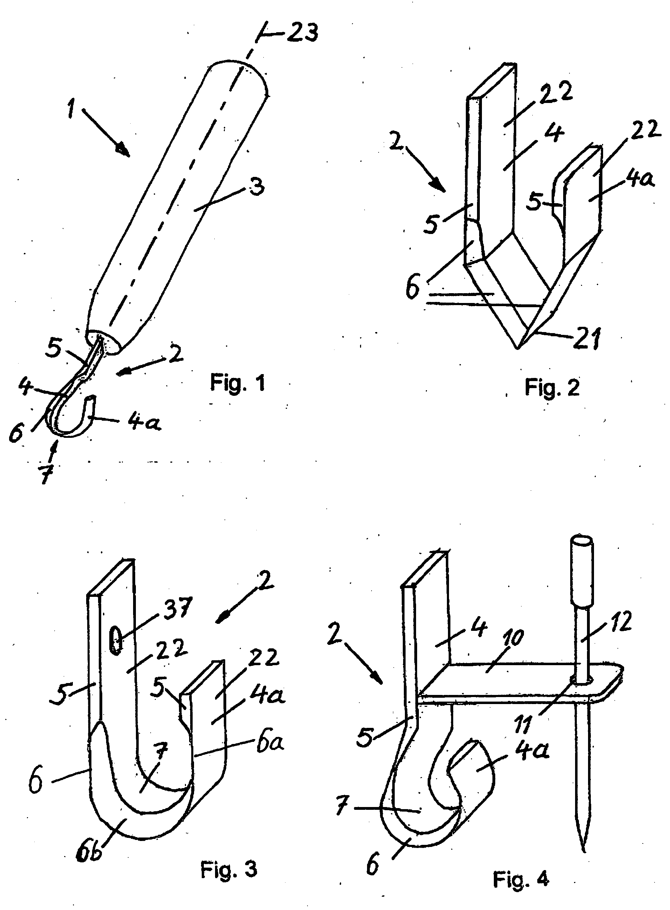

In Fig. 1 ist ein erstes Ausführungsbeispiel eines Excisionsskalpells 1 gemäß der Erfindung dargestellt. Das Excisionsskalpell weist ein Schneideteil 2 und ein Griffteil 3 auf, die am unteren oder distalen Ende des Griffteils 3 lösbar oder dauerhaft miteinander verbunden sind. Bei diesem Ausführungsbeispiel ist das Schneideteil 2 in seinem distalen Bereich mit Schneiden 6 versehen, die in je einem Schenkel 4, 4a, 6 ausgebildet sind. Die beiden, aus einem bandförmigen Material hergestellten und im Wesentlichen geraden Schenkel 4, 4a des Schneideteils 2 sind über einen ebenfalls mit einer Schneide versehenen, bogenförmigen Bereich oder Schenkel 7 miteinander verbunden, so dass sich eine in etwa tropfenförmige Kontur den Schneidenbereichs ergibt, wie dies in Fig. 4 im Detail gezeigt ist.

Eine Detailansicht eines alternativen Schneideteils 2 gemäß einem weiteren Ausführungsbeispiel ist in Fig. 2 dargestellt.

Aus Gründen der Übersichtlichkeit sind in dieser und in weiteren Figuren nicht sämtliche Einzelheiten mit Bezugszeichen versehen. Es sind im Hinblick auf den weitgehend symmetrischen Aufbau z.T. manche Bezugszeichen nur einmal eingetragen, obwohl zwei gleichartige Elemente vorhanden sind. Es sind jedoch alle Bezugszeichen eingefügt, die zur Erläuterung der als notwendig erachteten Einzelheiten nötig sind, wobei Bezugszeichen zur Bezeichnung gleicher baulicher Merkmale, die bereits in den Fig. 1 und 2 beschrieben wurden, weitgehend beibehalten wurden. Mit "distal" wird im Folgenden durchgehend derjenige Bereich eines Objekts bezeichnet, der vom Benutzer des Excisionsskalpells am weitesten entfernt ist. In diesem Bereich liegt beispielsweise die Spitze 21 des in Figur 2 gezeigten Schneideteils 2 oder das vordere, das Schneideteil 2 aufnehmende Ende 15 des Griffteils 3.Das in Fig. 2 gezeigte Schneideteil 2 eines erfindungsgemäßen Excisionsskalpells 1 weist verschieden lange Schenkel 4, 4a auf, die im distalen Bereich V-förmig, hier unter einem spitzen Winkel, angeordnet und miteinander verbunden sind. Der längere Schenkel 4 dient zur Verbindung mit dem hier nicht gezeigten Griffteil. Er weist bei diesem beispielhaften Ausführungsbeispiel einen Knick in seinem Verlauf auf, wie auch der kürzere Schenkel 4a. Die parallel verlaufenden Abschnitte 22 der Schenkel 4, 4a sind hier in nur teilweise mit Schneiden 6 versehen, was jedoch nicht zwingend ist. In einem weiteren hier nicht gezeigten Ausführungsbeispiel der Erfindung ist nur einer der beiden Schenkel (4, 4a) mit einem Knick versehen, während der andere Schenkel (4a, 4) durchgehend gerade verläuft. Welcher der beiden Schenkel (4, 4a) hierbei länger ausgebildet ist, als der andere, und zur Verbindung mit dem Griffteil dient, liegt im Belieben des Fachmanns.

Das Schneideteil 2 ist aus einem band- oder streifenförmigen Flachmaterial hergestellt, Als Material ist hier in beispielhafter Weise eine Edelstahllegierung vorgesehen. Die beiden Schenkel 4, 4a sind in der Spitze 21 miteinander verbunden, was bei einem metallischen Bandmaterial durch Knickung eines hinreichend langen Materialabschnitts erfolgen kann. Selbstverständlich sind auch andere Arten der Verbindung der beiden Schenkel 4, 4a denkbar, wie Laserschweißen, Löten oder Kleben. Hierbei ist zu beachten, dass sämtliche Schneiden 6 lückenlos aneinander stoßen und durch das Fügen nicht beschädigt werden.

Eine Vorderkante 5 jedes der Schenkel 4, 4a ist zumindest in dem der Spitze 21 benachbarten Bereich als Schneide 6 ausgebildet. Hierbei kann sich der als Schneide 6 ausgebildete Bereich auch über die gesamte Länge der beiden oder nur eines Schenkels 4, 4a erstrecken. Bei dem in Fig. 2 gezeigten Ausführungsbeispiel eines Schneideteils 2 weist nicht nur der V-förmige Klingenbereich eine Schärfe auf. An diesen Bereich schließt sich an jedem Schenkel ein weiterer, hier nur bereichsweise geschärfter Abschnitt 22 an, der gegenüber dem Klingenbereich in einem Winkel derart orientiert ist, dass diese Abschnitte 22 der beiden Schenkel 4, 4a parallel zueinander liegen. Der längere dieser Abschnitte 22 dient zur Befestigung oder Einbettung des Schneideteils 2 am oder im unteren Ende des Griffteils 3, was nachstehend noch erläutert wird.

Bei dem in Fig. 2 gezeigten Ausführungsbeispiel ist die vordere obere Kante des kürzeren Schenkels 4a abgerundet. Diese Maßnahme vermindert ein Verhaken des Schneideteils 2 und kann auch bei den übrigen freien Kanten der im Folgenden beschriebenen Schneideteile 2 angewendet werden.

Die mit einem Schneideteil 2 gemäß Fig. 2 erzielten Schnitte, die bevorzugt ziehend ausgeführt werden, ergeben eine streifen- oder fadenförmige, längliche Gewebeprobe mit einem dreieckigen Querschnitt, Sie hinterlassen eine entsprechend geformte Wunde geringer Breite, die, beispielsweise mittels eines Klammerpflasters, leicht zu schließen ist und eine kaum sichtbare Narbe hinterlässt.

Bei den nachfolgenden Ausführungsbeispielen sind analoge Teile mit gleichen Bezugszeichen versehen, so dass sich eine detaillierte Beschreibung dieser Teile erübrigt.

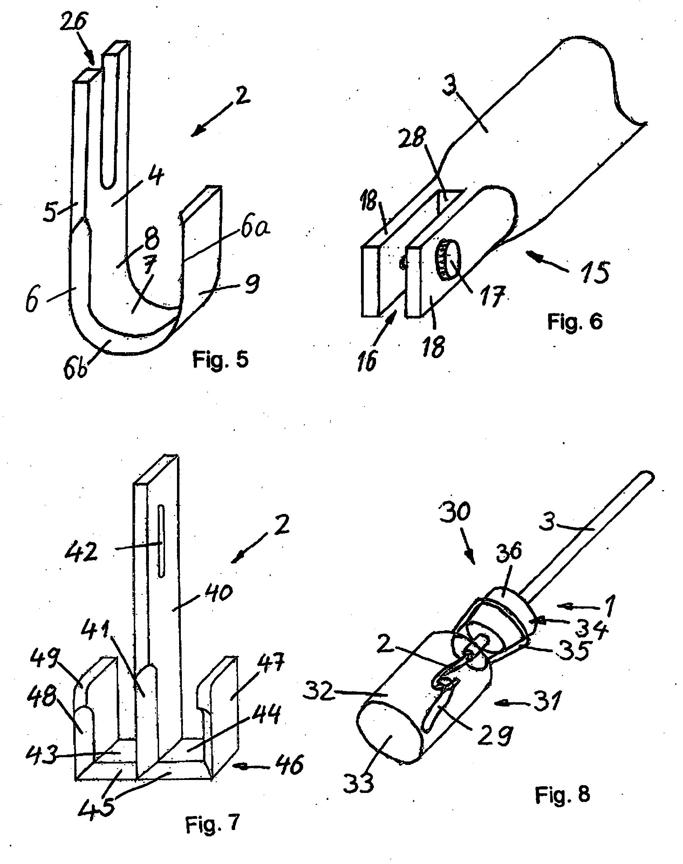

Die Fig. 3 eine Detailansicht eines weiteren Schneideteils 2 gemäß der Erfindung, bei dem die beiden Schenkel 4, 4a über einen gebogenen Bereich oder Schenkel 7 miteinander verbunden sind. Der gebogene Schenkel 7 ist, genauso wie die an ihn anschließenden Bereiche der Schenkel 4, 4a des Schneideteils 2 mit einer Schneide 6 versehen, wobei die Schärfen aller Schenkel 4, 4a, 7 lückenlos ineinander übergehen. Es versteht sich, dass sich die Schneiden 6 auch über die gesamte Länge der Vorderkante 5 des Schneideteils erstrecken können. Wie bei dem Ausführungsbeispiel gemäß Fig. 2 dienen auch bei dieser Bauform des Schneideteils 2, der obere Abschnitt 22 des längeren Schenkels 4 zur Einbettung oder sonstigen Befestigung des Schneideteils 2 am Griffteil 3. Hierfür sind im längeren Schenkel 4 Durchbrüche oder Bohrungen 37 vorgesehen, die dem Durchtritt einer Fixierschraube 17 (siehe Fig. 6) oder von Einbettungsmaterial dienlich sind

Die mit einem Schneideteil gemäß Fig. 3 erzielten Schnitte, die bevorzugt ziehend ausgeführt werden, ergeben eine streifenförmige, längliche Gewebeprobe mit einem U-förmigen Querschnitt, Sie hinterlassen eine entsprechend geformte Wunde geringer Breite, die, wie zuvor beschrieben, versorgt werden kann und die zu einer ästhetisch nicht zu beanstandenden, dünnen Narbe abheilt.

In Fig. 4 eine Detailansicht eines dritten erfindungsgemäßen Schneideteils 2 mit einem seitlichen Ansatzteil 10 dargestellt. Das Ansatzteil 10 erstreckt sich in einem rechten Winkel zur Längsachse 23 des Griffteils 2 oder zur Ebene des langen geraden Abschnitts des längeren Schenkels 4, wie in Fig. 4 dargestellt ist. Das Ansatzteil 10 ist auch in einem rechten Winkel in Bezug auf die Schnittrichtung, die durch die Orientierung der Schneiden 6 vorgegeben ist. Das Ansatzteil 10 ist in seinem vom Schneideteil 2 bzw. Griffteil 3 entfernten Bereich mit einer durchgehenden Bohrung 11 versehen, die dem Durchtritt einer Nadel 12 dienlich ist. Diese Nadel 12 erlaubt es das Excisionsskalpell 1 nach Art eines Zirkels einzusetzen, wobei die Nadel 12 als Drehachse dient. Hierdurch ist es möglich, Gewebeproben aus einem kreisbogenförmigen Bereich der Haut oder eines anderen Gewebebereiches zu gewinnen.

Das Schneideteil 2 gemäß Fig. 4 stellt eine Abwandlung des in Fig. 3 gezeigten Ausführungsbeispiels dar, da hier die oberen Abschnitte 22 der Schenkel 4, 4a nicht parallel sind sondern sich einander annähern. sind, so dass sich insgesamt eine Tropfenform des Schneideteils 2 ergibt. Eine Tropfenform des Schneideteils 2 ist auch in Figur 1 gezeigt, hier jedoch ohne Ansatzteil.

Bei einer Einbettung in Kunststoff oder ein anderes Material sind die einzubettenden Bereiche des Schneideteils, d.h. der proximale Teil des längeren Schenkels 4 bevorzugt mit Durchbrüchen (26, 37) (s. Fig. 3, 6 und 7) versehen, durch welche das Eingußmaterial hindurchtreten kann. Dies bewirkt eine besonders gute Fixierung des Schneideteils 2 im Griffteil 3. Derartige Durchbrüche können auch dem Durchtritt einer Fixierschraube 17 dienen, wenn ein Schneideteil 2 mit einem Griffteil 3 in lösbarer Weise verbunden werden soll. Ein hierfür geeignetes Griffteil 3 ist in beispielhafter Weise in Figur 6 dargestellt.

Fig. 5 zeigt eine Detailansicht eines alternativen Schneideteils 2, bei dem ein gerader Schenkel 8 mit einem gebogenen Schenkel 9 verbunden ist. Bei dieser Bauform ist gemäß der Erfindung nur der gerade längere Schenkel 8 mit einem Griffteil 3, hier nicht gezeigten Griffteil 3 verbunden. Der gerade Schenkel 8 weist bei diesem Ausführungsbeispiel in seinem oberen Bereich einen Schlitz 26 auf, der zur Verbesserung der Fixierung an einem Griffteil 3 dienlich ist, wie anhand der Fig. 6 näher erläutert wird.

Die Fig.6 zeigt eine Detailansicht eines vorderen, distalen Bereichs 15 eines Griffteils 3 gemäß der Erfindung. In diesem Bereich 15 ist ein Schlitz 16 ausgebildet, der sich in Längsrichtung des generell stabförmigen Griffteils 3 erstreckt. Der Bereich des Schlitzes 16 definiert zwei Klemmbacken 18, zwischen die das obere Ende des Schneideteils2 mit dem Abschnitt22 des längeren Schenkels 4 in den Schlitz 16 einführbar ist. Die Klemmbacken 18 sind mittels einer Fixierschraube 17, die quer zum Schlitz 16 diesen durchsetzt, und in einem Gewinde im Griffteil 3 oder einer Gewindemutter auf dessen Gegenseite zusammenwirkt, aufeinander zu bewegbar. Hierdurch ist eine lösbare Fixierung des Schneideteils 2 im Griffteil 3 mittels Klemmung erzielbar.

Die Sicherheit der Fixierung kann dadurch unterstützt werden, dass in dem Abschnitt 22 des längeren Schenkels 4 ein Längsschlitz 26 ausgebildet ist, wie dies beispielhaft in Figur 5 bei einem geraden Schenkel 8 gezeigt ist. Der Längsschlitz 26 ist hierfür auf den Durchmesser des Gewindebereichs der Stellschraube 17 abgestimmt, so dass der Abschnitt 22 bis zur Rückwand 28 des Schlitzes 16 geschoben werden kann und an der Rückwand 28 anliegt.

In Figur 7 ist ein spezielles Ausführungsbeispiel der Erfindung gezeigt, dessen Schneideteil 2 ankerförmig ausgebildet ist. Dieses Schneideteil 2 weist einen langen, geraden ersten Schenkel 40 auf, der in seinem distalen, unteren Bereich mit einer Schneide 41 versehen ist. Im oberen Bereich des Schenkels 40 ist ein länglicher Durchbruch 42 angeordnet, der dem Durchtritt von Einbettungsmaterial oder von einer Fixierschraube 17 dienen kann, wie zuvor beschrieben. Am unteren Ende des ersten Schenkels 40 sind spiegelsymmetrisch zur Ebene dieses Schenkels 40 zwei weitere Schenkel 43 angeordnet und unlösbar mit dem ersten Schenkel 40 verbunden, was beispielsweise durch Laserschweißen bewirkbar ist. Die beiden weiteren Schenkel 43 stehen hier beispielhaft in einem ersten, der Verbindungsstelle mit dem ersten Schenkel 40 benachbarten ersten Abschnitt 44 senkrecht zum ersten Schenkel 40. Sie weisen in diesem ersten Abschnitt 44 je eine Schneide 45 an der Vorderseite auf, die lückenlos mit der Schneide 41 des ersten Schenkels41 zusammenstoßen. Im weiteren Verlauf der weiteren Schenkel 43 weisen diese einen hier beispielhaft rechtwinkligen Knick 46 auf, der jeweils einen zweiten Abschnitt 47 der weiteren Schenkel 43 begrenzt. Die zweiten Abschnitte 47 weisen jeweils ebenfalls eine Schneide 48 auf, deren Erstreckung gleich oder geringer ist, als der Schneidenbereich des ersten Schenkels 40.

Es versteht sich und ist vom Erfindungsgedanken umfasst, dass der Winkel des Knicks 45 auch von 90° abweichen kann. Die zweiten Abschnitte 47 der Schenkel 43 stehen dann nicht parallel zueinander, was beim Einsatz eines derart bestückten Excisionsskalpells 1 Gewebeproben mit einem trapezförmigen Querschnitt ergibt.

Bei dem in Figur 7 gezeigten Ausführungsbeispiel sind die oberen Ecken 49 der zweiten Abschnitte 47 gerundet, was ein Verhaken reduzieren kann.

In einer nicht gezeigten Abwandlung des in Figur 7 gezeigten Ausführungsbeispiels sind die beiden weiteren Schenkel 43 des Schneideteils 2 gebogen anstatt geknickt, so dass die generelle Ankerform des Schneideteils 2 gegeben ist. Mit Schneideteilen 2 dieser Bauform werden mit einem Schnittvorgang gleichzeitig zwei getrennte Gewebeproben mit einem U-förmigen Querschnitt aus einem bestimmten Gewebebereich erzeugt. Dies kann bei bestimmten Anwendungen erwünscht sein, beispielsweise, wenn die Biopsieprobe mit verschiedenen Methoden untersucht werden soll.

Es versteht sich, dass von einer Bauform des Schneideteils 2, wie sie in Figur 7 gezeigt ist, abgewichen werden kann, ohne den Erfindungsgedanken zu verlassen. So ist beispielsweise eine unsymmetrische Ausbildung des Schneideteils 2 denkbar, in der die beiden weiteren Schenkel 43 unterschiedlich geformt und dimensioniert sein können.

In Fig. 8 ist eine Excisionseinheit 30 gemäß der Erfindung in einem zusammengefügten Zustand dargestellt. Die Excisionseinheit 30 umfasst ein Excisionsskalpell 1 gemäß der Erfindung mit einem Schneideteil 2 und einem Griffteil 3 sowie ein Behälterteil 31 mit einem Grundkörper 32. Der hier beispielhaft als Hohlzylinder gezeigte Grundkörper 32 ist an der Unterseite mit einem Boden 33 verschlossen und weist an der Oberseite eine Öffnung 34 auf, die hier beispielhaft in einem Ansatz 35 geringeren Durchmessers ausgebildet ist. Das Griffteil 3 des Excisionsskalpells 1 weist bei diesem beispielhaften Ausführungsbeispiel einen Bereich 36 auf, der gegenüber dem übrigen Teil des Griffteils 3 derart verdickt ist, dass er nach Art eines Stopfens abdichtend in die Öffnung 34 des Behälterteils 31 einsetzbar ist. Der Bereich ist hierfür in der Form und in seinen Abmessungen an die Öffnung 34 angepasst. In einer bevorzugten Ausführungsform ist das Griffteil 3 zweiteilig ausgebildet. Ein erster Teil wird hierbei durch den verdickten Bereich 36 gebildet an dem das Schneideteil 3 fixiert ist. Der andere, zweite Teil ist stabförmig und ist in einer Sackbohrung im ersten Teil lösbar fixiert, was durch Klemmung bei entsprechend angepassten Durchmessern von Sackbohrung und zweitem Teil erfolgen kann. Auch eine konische Ausbildung der beiden Kontaktbereiche ist denkbar.

Alternativ kann die abdichtende Verbindung zwischen dem Behälterteil 31 und dem Griffteil 3 des Excisionsskalpells durch eine Verschraubung erfolgen, wofür die beiden Teile mit komplementären Gewinden und bevorzugt mit elastischen Dichtringen oder Dichtlippen versehen sind. Auch eine Verbindung mittels eines Bajonettverschlusses ist denkbar.

Die Excisionseinheit 30 kann in der Praxis steril verpackt auf Vorrat gehalten werden, wobei das Behälterteil 31 bereits mit Formalin oder einer anderen geeigneten Flüssigkeit gefüllt und mit dem Griffteil 3 verschlossen sein kann. Sämtliche Teile sind hierfür bevorzugt mit einer Kodierung versehen, welche eine spätere Zuordnung der Gewebeprobe sicherstellen kann. Als Kodierung sind beispielsweise Nummern- oder Strichkodes denkbar, die auf die einzelnen Komponenten mittels Etiketten aufgebracht sind oder auf sie aufgedruckt oder eingebrannt oder eingeprägt sind.

Nach Durchführung der Biopsie wird das Excisionsskalpell 1 mit der am Schneideteil 2 anhaftenden Gewebeprobe 29 in das Behälterteil 30 eingeführt und mittels des verdickten Bereichs 36 flüssigkeitsdicht verschlossen. Danach kann der lösbare zweite Teil des Griffteils 2 entfernt werden. Die derart bestückte Excisionseinheit 30 ist damit für den Versand zur Untersuchung an ein Labor bereit.

| | | | |
|---|---|---|---|
| Bezugszeichenliste | | 26 | Längsschlitz |
| 1 | Excisionsskalpell | 28 | Rückwand |
| 2 | Schneideteil | 29 | Gewebeprobe |
| 3 | Griffteil | 30 | Excisionseinheit |
| 4, 4a | Schenkel | 31 | Behälterteil |
| 5 | Vorderkante. | 32 | Grundkörper |
| 6 | Schneide | 33 | Boden |
| 6a, 6b | Schneide | 34 | Öffnung |
| 7 | gebogener Schenkel | 35 | Ansatz |
| 8 | gerader Schenkel | 36 | Bereich |
| 9 | gebogener Schenkel | 37 | Bohrung |
| 10 | Ansatz | 40 | erster Schenkel |
| 11 | Durchbruch | 41 | Schneide |
| 12 | Nadel | 42 | Durchbruch |
| 15 | distales Ende | 43 | weiterer Schenkel |
| 16 | Schlitz | 44 | erster Abschnitt |
| 17 | Stellschraube | 45 | Schneide |
| 18 | Klemmbacken | 46 | Knick |
| 21 | Spitze | 47 | zweiter Abschnitt |
| 22 | Abschnitt | 48 | Schneide |
| 23 | Längsachse | 49 | obere Ecke |

## Patentansprüche

**1.** Excisionsskalpell (1) mit einem Schneideteil (2), das mit einem Griffteil (3) verbunden ist, zur Gewinnung von streifen- oder fadenförmigen, länglichen Gewebeproben für histologische Untersuchungen mittels eines ziehenden Schnitts
**dadurch gekennzeichnet,**
- **dass** das Schneideteil (2) mindestens zwei mit einander verbundene, flache Schenkel (4, 4a) aufweist, die an einer Vorderkante (5) zumindest bereichsweise je eine Schneide (6, 6a) aufweisen und
- **dass** nur einer der Schenkel (4,4a) mit dem Griffteil (3) verbunden ist.

**2.** Excisionsskalpell gemäß Anspruch 1, **dadurch gekennzeichnet, dass** das Schneideteil (2) zwei verschieden lange Schenkel (4, 4a) aufweist, die am distalen, apikalen Ende des Schneideteils (2) unter einem Winkel zueinander angeordnet sind und in der Spitze (21) des Winkels lückenlos ineinander übergehen, wobei der längere der beiden Schenkel (4, 4a) mit dem Griffteil verbunden ist.

**3.** Excisionsskalpell gemäß Anspruch 2, **dadurch gekennzeichnet, dass** zumindest einer der Schenkel (4, 4a) zumindest einen von der Spitze (21) des Winkels beabstandeten Knick aufweist.

**4.** Excisionsskalpell gemäß Anspruch 1, **dadurch gekennzeichnet, dass** das Schneideteil (2) eine U-Form oder Tropfenform mit zwei geraden Schenkeln (4, 4a) und einen die Schenkel (4, 4a) verbindenden, gebogenen Schenkel (7) aufweist, wobei auch der gebogene Schenkel (7) eine Schneide (6b) aufweist, die lückenlos in die Schneiden (6, 6a) der geraden Schenkel (4,4a) übergeht

**5.** Excisionsskalpell gemäß Anspruch 1, **dadurch gekennzeichnet, dass** das Schneideteil (2) einen geraden Schenkel (8) und einen gebogenen Schenkel (9) aufweist, deren Schneiden (6) lückenlos ineinander übergehen, wobei nur der gerade Schenkel (8) mit dem Griffteil (3) verbunden ist.

**6.** Excisionsskalpell gemäß Anspruch 1, **dadurch gekennzeichnet, dass** das Schneideteil (2) einen geraden Schenkel (40) aufweist, an dessen distalen Endbereich zwei weitere, gerade, gebogene oder geknickte Schenkel (43) unter einem Winkel zwischen 10° und 90° angeordnet sind, wobei sämtliche Schenkel (40,43, 47) zumindest bereichsweise mit Schneiden (41, 45, 48) versehen sind, die im distalen Bereich des geraden Schenkels (40) lückenlos ineinander übergehen.

**7.** Excisionsskalpell gemäß einem der voranstehenden Ansprüche, **dadurch gekennzeichnet, dass** am Griffteil (3) oder am Schneideteil (2) ein seitliches Ansatzteil (10) angeordnet ist, das sich rechtwinklig zur Schnittrichtung und zur Längsachse (23) des Griffteils (3) erstreckt, wobei das Ansatzteil (10) in seinem vom Schneideteil (2) oder vom Griffteil (3) entfernten Bereich einen Durchbruch oder eine durchgehende Bohrung (11) aufweist.

**8.** Excisionsskalpell gemäß einem der Ansprüche 1 bis 7, **dadurch gekennzeichnet, dass** das Schneideteil (2) lösbar und fixierbar mit dem Griffteil (3) verbunden sind.

**9.** Excisionsskalpell gemäß einem der Ansprüche 1 bis 7, **dadurch gekennzeichnet, dass** das Schneideteil (2) unlösbar mit dem Griffteil (3) verbunden ist.

**10.** Excisionsskalpell gemäß einem der Ansprüche 1 bis 9, **dadurch gekennzeichnet, dass** das Schneideteil (2) aus einem biegbaren und/oder flexiblen Bandmaterial hergestellt ist, von dem zumindest eine Seitenkante als eine Schneide (6) ausgebildet ist.

**11.** Excisionsskalpell gemäß Anspruch 10, **dadurch gekennzeichnet, dass** das biegbare Band aus rostfreiem Stahl hergestellt ist.

**12.** Excisionsskalpell gemäß einem der voranstehenden Ansprüche, **dadurch gekennzeichnet, dass** das Griffteil (3) stabförmig und zumindest bereichsweise abgeflacht und/oder an der Oberfläche strukturiert ist.

**13.** Excisionsskalpell gemäß einem der voranstehenden Ansprüche, **dadurch gekennzeichnet, dass** das Griffteil (3) und das Schneideteil (2) aus einem sterilisierbaren Material bestehen..

**14.** Excisionsskalpell gemäß einem der voranstehenden Ansprüche, **dadurch gekennzeichnet, dass** das Griffteil (3) Mittel zum Fixieren eines Schenkels (4) des Schneideteils (2) aufweist.

**15.** Excisionsskalpell gemäß einem der voranstehenden Ansprüche, **dadurch gekennzeichnet, dass** das Griffteil (3) in dem Schneideteil (2) benachbarten Bereich eine Sollbruchstelle aufweist.

**16.** Excisionsskalpell gemäß einem der voranstehenden Ansprüche, **dadurch gekennzeichnet, dass** das Griffteil (3) zweiteilig ausgebildet ist, und dass beide Teile lösbar miteinander verbunden sind

**16.** Excisionsskalpell gemäß einem der voranstehenden Ansprüche, **dadurch gekennzeichnet, dass** das Griffteil (3) Mittel zum Beleuchten der Schnittstelle aufweist.

**17.** Excisionsskalpell gemäß einem der voranstehenden Ansprüche, **dadurch gekennzeichnet, dass** der Abstand zwischen zwei geraden Schenkeln (4, 4a) entgegen der Schnittrichtung größer wird.

**18.** Excisionsskalpell gemäß einem der voranstehenden Ansprüche, **dadurch gekennzeichnet, dass** der Abstand zwischen zwei Schenkeln (4, 4a) zumindest bereichsweise veränderbar ist.

**19.** Excisionseinheit (30) mit einem Excisionsskalpell (1) gemäß einem der voranstehenden Ansprüche, **dadurch gekennzeichnet, dass** die Excisionseinheit (30) ein Behälterteil (31) aufweist, welches mit dem Excisionsskalpell (1) in lösbarer Weise verbindbar ist.

**20.** Excisionseinheit gemäß Anspruch 19, **dadurch gekennzeichnet, dass** das Behälterteil (31) rohrförmig mit einem verschlossenen Boden (32) und mit einer dem Boden (32) gegenüberliegenden Öffnung (33) ausgebildet ist, in welche der distale Bereich des Excisionsskalpells (1) mit dem Schneideteil (2) einführbar und fixierbar ist.

**21.** Excisionseinheit gemäß Anspruch 20, **dadurch gekennzeichnet, dass** das Griffteil (3) des Excisionsskalpells (1) einen Bereich (34) aufweist, dessen Form und Dimensionen derart an die Form und Größe der Öffnung (33) des Behälterteils (31) abgestimmt ist, dass das Excisionsskalpell (1) in die Öffnung (33) des Behälterteils (31) nach Art eines Stopfens oder mittels eines Gewindes oder Bajonettverschlusses abdichtend einsetzbar ist.
